(19) Europäisches Patentamt
European Patent Office
Office européen des brevets

(11) Numéro de publication: **0 209 770 A1**

(12) # DEMANDE DE BREVET EUROPEEN

(21) Numéro de dépôt: 86109150.2

(22) Date de dépôt: 04.07.86

(51) Int. Cl.⁴: C 07 C 69/606
C 07 C 103/58, C 07 C 103/60
C 07 D 295/18, A 61 K 31/23
A 61 K 31/16, A 61 K 31/40

(30) Priorité: 05.07.85 FR 8510363

(43) Date de publication de la demande:
28.01.87 Bulletin 87/5

(84) Etats contractants désignés:
AT BE CH DE FR GB IT LI NL SE

(71) Demandeur: CENTRE INTERNATIONAL DE RECHERCHES DERMATOLOGIQUES C.I.R.D. Groupement d'Intérêt Economique dit:
Sophia Antipolis
F-06560 Valbonne(FR)

(72) Inventeur: Shroot, Braham
Villa 35 Hameaux de Val Bosquet
F-06600 Antibes(FR)

(72) Inventeur: Hensby, Christopher
89, Chemin d'Andon villa Madru
F-06410 Biot(FR)

(72) Inventeur: Maignan, Jean
8, rue Halévy
F-93290 Tremblay les Gonesses(FR)

(72) Inventeur: Lang, Gérard
44, Avenue Lacour
F-95210 saint Gratien(FR)

(72) Inventeur: Restle, Serge
149, Rue Anatole France
F-93601 Aulnay-sous-Bois(FR)

(72) Inventeur: Colin, Michel
10, Allée Cécile
F-93190 Livry-Gargan(FR)

(74) Mandataire: Casalonga, Axel et al,
BUREAU D.A. CASALONGA OFFICE JOSSE & PETIT
Morassistrasse 8
D-8000 München 5(DE)

(54) **Esters et amides de l'acide eicosatriynoique et leur application en pharmacie et en cosmétique.**

(57) Composé caractérisé par le fait qu'il répond à la formule:

$$C_8H_{17}-(C\equiv C-CH_2)_3-CH_2CH_2COR \quad (I)$$

dans laquelle R est un groupement alcoxy inférieur en $C_1$-$C_8$ ou cycloalcoxy en $C_4$-$C_8$, substitués par un ou plusieurs groupements hydroxyles et/ou interrompu par un ou plusieurs hétéroatomes choisis parmi l'oxygène et le soufre, un groupement amino de structure 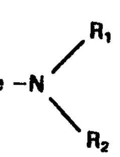

dans laquelle $R_1$ ou $R_2$ identiques ou différents désignent un atome d'hydrogène, un radical alkyle inférieur en $C_1$-$C_8$ linéaire ou ramifié, éventuellement interrompu par un ou plusieurs hétéroatomes choisis parmi l'oxygène, le soufre, l'azote, ce radical alkyle pouvant être substitué par un ou plusieurs groupements hydroxyles, $R_1$ et $R_2$ ne pouvant désigner simultanément hydrogène ou bien $R_1$ et $R_2$ forment ensemble avec l'atome d'azote, un hétérocycle contenant éventuellement comme hétéroatome supplémentaire de l'oxygène, du soufre ou de l'azote, l'un des radicaux $R_1$ ou $R_2$ pouvant encore désigner, lorsque l'autre est un atome d'hydrogène, un radical aryle de formule (II) :

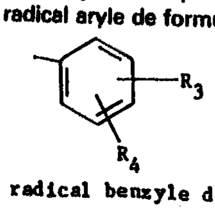

(II)

ou bien un radical benzyle de formule (III) :

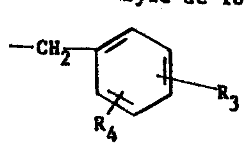

(III)

./...

EP 0 209 770 A1

dans lesquelles $R_3$ et $R_4$ désignent, indépendamment l'un de l'autre un atome d'hydrogène, un radical alkyle en $C_1$-$C_4$, un groupement hydroxyle, un atome d'halogène, un groupement carboxyle ou trifluorométhyle, la fonction amine pouvant également provenir d'un sucre ainsi que leurs isomères et sels pharmaceutiquement et cosmétiquement acceptables.

Esters et amides de l'acide eicosatriynoïque et leur application en pharmacie et en cosmétique.

---

La présente invention a pour objet de nouveaux composés constitués par des esters et des amides de l'acide eicosatriynoïque ainsi que leur application d'une part comme agent thérapeutique dans le traitement ou la prophylaxie des maladies allergiques et dans le traitement des dermatoses et des maladies inflammatoires et d'autre part dans des compositions cosmétiques.

Il est connu qu'un certain nombre de substances jouent un rôle important dans le processus inflammatoire de la peau tels que l'acné, les dermatoses, comme par exemple le psoriasis, l'eczéma etc.. Ces substances parmi lesquelles les prostaglandines, les acides hydroxyeicosatétraénoïques, les thromboxanes et les leucotriènes, ont toutes une origine commune qui est l'acide arachidonique. (Voir "VOORHEES-Leukotrienes and Other Lipoxygenase Products in the Pathogenesis and Therapy of Psoriasis and Other Dermatoses" Arch Dermatol, Vol. 119, Juillet 1983, 541-547).

La formation de ces substances résulte essentiellement de la libération de l'acide arachidonique lié par une liaison ester aux lipides présents dans l'épiderme (par exemple les phospholipides).

On a déjà préconisé antérieurement, pour le traitement de maladies de la peau, soit des inhibiteurs de la cyclooxygénase empêchant la formation des prostaglandines tels que l'indométhacine, la vitamine E, etc.; ou alors des substances susceptibles d'inhiber les lipoxygénases tels que l'acide eicosatétraynoïque.

On a déjà préconisé pour le traitement du psoriasis l'acide eicosa tétraynoïque-5,8,11,14 ainsi que l'acide eicosa triynoïque-5,8,11 et leurs esters d'alkyle inférieurs. (US-A-4 190 669).

La demanderesse a découvert, de façon surprenante, que des esters ou amides particuliers de l'acide eicosatriynoïque-5,8, 11 inhibaient le métabolisme enzymatique de l'acide arachidonique provoqué par la cyclo-oxygénase et les lipoxygénases. Ce résultat est particulièrement inattendu en raison du blocage de la fonction acide sous la forme des esters ou amides définis ci-dessous.

Ces composés présentent par ailleurs une biodisponibilité différente de celle des acides correspondants leur conférant des propriétés thérapeutiques améliorées.

La présente invention a donc pour objet les nouveaux dérivés de l'acide eicosatriynoïque-5,8,11.

Un autre objet de l'invention est constitué par les compositions pharmaceutiques contenant comme substance active de tels composés.

Un autre objet de l'invention est également constitué par le procédé de préparation de ces dérivés.

L'invention a également pour objet l'utilisation dans le domaine de la cosmétique, de ces composés, notamment dans des compositions antiacnéiques, solaires ou postsolaires ou dans le traitement des dermatites séborrhéiques.

D'autres objets de l'invention apparaîtront à la lecture de la description et des exemples qui suivent.

Les composés conformes à l'invention sont essentiellement caractérisés par le fait qu'ils répondent à la formule :

$$C_8H_{17}\text{—}(C\equiv C-CH_2)_3\text{—}CH_2CH_2COR \qquad (I)$$

dans laquelle :

R est un groupement alcoxy inférieur en $C_1$-$C_8$ ou cycloalcoxy en $C_4$-$C_6$, substitués par un ou plusieurs groupements hydroxyles et/ou interrompu par un ou plusieurs hétéroatomes choisis parmi l'oxygène et le soufre, ou bien un

groupement amino de structure $-N\begin{matrix}R_1\\R_2\end{matrix}$ dans laquelle $R_1$ et $R_2$

peuvent être identiques ou différents et correspondre à un atome d'hydrogène, un radical alkyle inférieur en $C_1$-$C_8$ linéaire ou ramifié, un radical alkyle inférieur en $C_1$-$C_8$ interrompu par un ou plusieurs hétéroatomes choisis parmi l'oxygène, l'azote ou le soufre et/ou substitué par un ou plusieurs groupements hydroxyle, $R_1$ et $R_2$ ne désignent pas simultanément un atome d'hydrogène, $R_1$ et $R_2$ peuvent également former, avec l'atome d'azote, un hétérocycle comportant éventuellement comme hétéroatome supplémentaire l'azote, l'oxygène ou le soufre;

l'un des radicaux $R_1$ ou $R_2$ peut également désigner, lorsque l'autre est un atome d'hydrogène, un radical aryle de formule (II) :

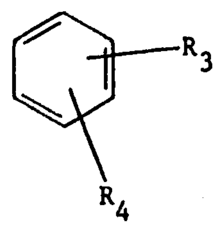

(II)

ou bien un radical benzyle de formule (III) :

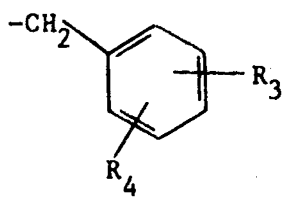

(III)

dans lesquelles $R_3$ et $R_4$ représentent, indépendamment l'un de l'autre, un atome d'hydrogène, un radical alkyle en $C_1$-$C_4$, un groupement hydroxyle, un atome d'halogène tel que chlore, brome ou fluor, un groupement carboxyle ou un groupement trifluorométhyle,

le groupement amino peut également provenir d'un sucre tel que de préférence la glucosamine

ainsi que leurs isomères et les sels pharmaceutiquement et cosmétiquement acceptables comme les sels d'acide minéraux ou organiques tels que les acides chlorhydrique, lactique, tartrique et citrique.

Les composés particulièrement préférés de l'invention sont choisis parmi les composés suivants :

- lorsque R désigne un groupement alcoxy en $C_1$-$C_8$ substitué, il désigne plus particulièrement un groupement hydroxy-2 éthoxy, hydroxy-2 propyloxy, dihydroxy-2,3 propyloxy ou encore son isomère, le radical dihydroxy-1,3 propyloxy-2.

- lorsque R désigne un groupement amino de structure 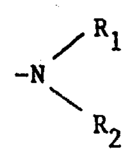

les significations préférées de $R_1$ et $R_2$ sont les suivantes :

. pour $R_1$ et/ou $R_2$ alkyle inférieur, méthyle, éthyle, isopropyle,

. pour $R_1$ et/ou $R_2$ alkyle interrompu par un ou plusieurs hétéroatomes tel que $-CH_2-CH_2-O-CH_2CH_2OH$

. pour $R_1$ et/ou $R_2$ alkyle en $C_1$-$C_8$ substitué par un ou plusieurs groupements hydroxyles, les significations préférées sont les suivantes :

$CH_2CH_2OH$, $CH_2CHOHCH_3$, $CH_2CHOHCH_2OH$

. lorsque $R_1$ et $R_2$ forment un cycle, les composés préférés de l'invention sont des amides de la pyrrolidine, de la morpholine, de la pipérazine et de l'hydroxy-2' éthyl-4 pipérazine.

. lorsque l'un des radicaux $R_1$ ou $R_2$ représente H et l'autre un radical aryle, il s'agit du radical parahydroxyphényle.

Parmi les composés de formule I, on peut citer :

- le dihydroxy-2',3' propyl eicosatriynoate-5,8,11
- le N-(hydroxy-2 éthyloxyéthyl) eicosatriynamide-5,8,11
- le N-éthyl eicosatriynamide-5,8,11
- le pyrrolidino eicosatriynamide-5,8,11
- le N,N-di(hydroxy-2 éthyl)eicosatriynamide-5,8,11
- le N-(hydroxy-2 éthyl)eicosatriynamide-5,8,11
- le N-(dihydroxy-2,3 propyl)eicosatriynamide-5,8,11
- l'(hydroxy-2 éthyl)-4' pipérazino eicosatriynamide-5,8,11
- le N-(parahydroxyphényl)eicosatriynamide -5,8,11.

Parmi les composés préférés, on peut citer :

- le N-(hydroxy-2 éthyloxyéthyl)eicosatriynamide-5,8,11
- le dihydroxy-2',3' propyl eicosatriynoate-5,8,11
- le N-éthyl eicosatriynamide-5,8,11
- le pyrrolidino eicosatriynamide-5,8,11
- l'(hydroxy-2 éthyl)-4' pipérazino eicosatriynamide-5,8,11.

Ces esters ou amides sont préparés à partir de l'acide eicosatriynoïque-5,8,11 connu en lui-même depuis longtemps (voir A. VAN DORP et Coll., Recueil <u>85</u>, 1966, page 1233). Le schéma A ci-dessous décrit cette synthèse. La synthèse du produit marqué au $^{14}C$ ainsi que la synthèse de nombreux homologues de cet acide sont décrites en particulier dans la thèse de "ULLMAN MYRON, 1970, Ohio State University, Ph.D., Biochemistry".

L'acide est décrit, par ailleurs, dans le brevet américain US-A 3 033 884.

La demanderesse a également découvert, ce qui constitue un autre objet de l'invention, un nouveau procédé de préparation de l'acide eicosatriynoïque-5,8,11 qui permet de produire les esters et amides conformes à l'invention avec des rendements et des coûts plus avantageux (schéma B).

Ce procédé est essentiellement caractérisé par le fait que l'on synthétise l'halogéno-1 tétradécadiyne-2,5 (3) en une seule étape en faisant réagir le décyne-1 (1) avec le dihalogéno-1,4 butyne-2 (2) en présence d'une base forte telle qu'un organo magnésien. Au cours de cette réaction, l'acétylure du décyne (1) est formé par échange avec cette base forte, puis est mis en réaction avec le dihalogénure (2) en excès, et l'on obtient d'une façon surprenante le composé (3) avec un très bon rendement. Le résultat est particulièrement surprenant lorsque l'on sait que la synthèse de cet halogénure était jusqu'à présent obtenue en mettant en oeuvre des réactions d'allongement de chaîne à l'aide d'un dérivé d'alcool propargylique avec des rendements relativement faibles.

Schéma réactionnel A

a) Synthèse du bromo-1 tétradécadiyne-2,5

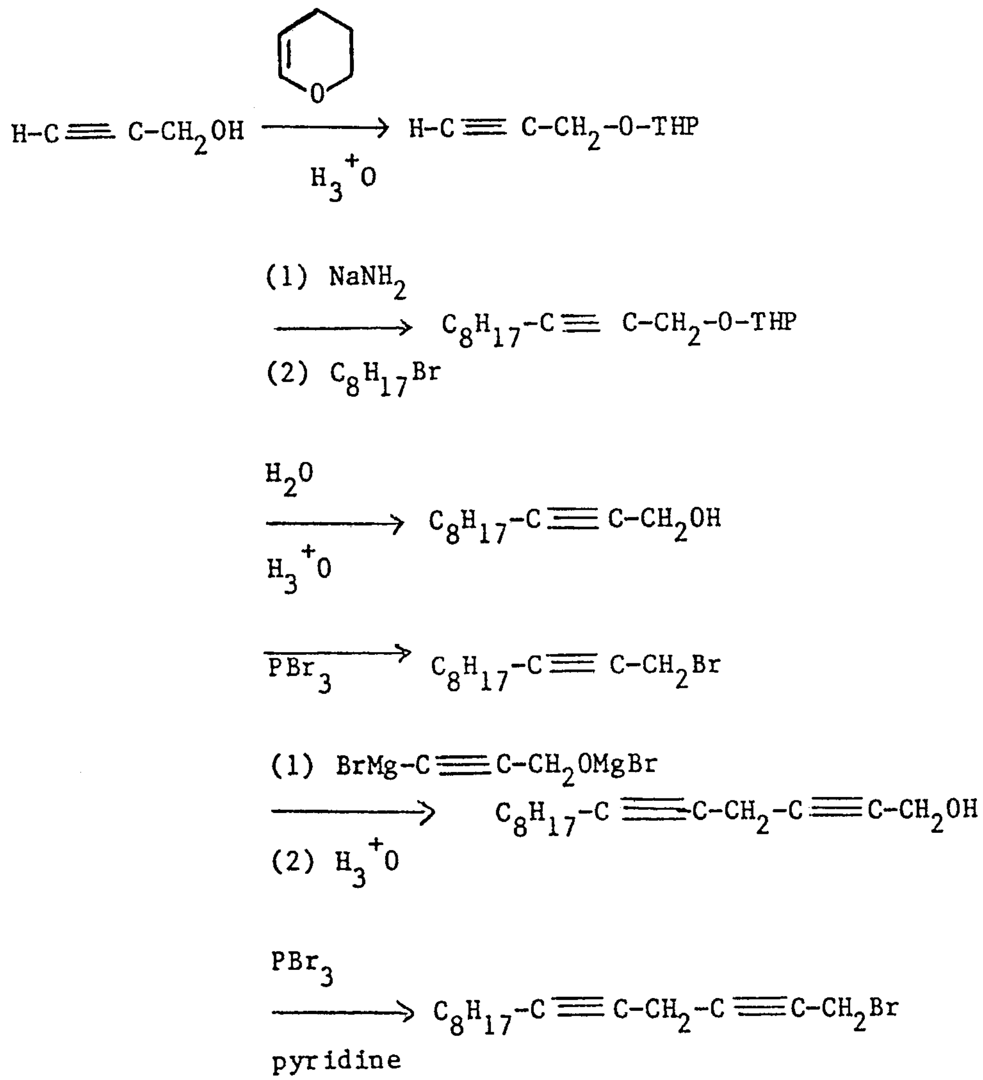

(b) Synthèse de l'acide hexyne-5 carboxylique

$$Br(CH_2)_3Cl \xrightarrow{NaC\equiv CH} H\text{-}C\equiv C\text{-}(CH_2)_3Cl$$

$$\xrightarrow{NaCN} H\text{-}C\equiv C\text{-}(CH_2)_3CN$$

$$\xrightarrow[(2)\ H_3^+O]{(1)\ KOH} H\text{-}C\equiv C\text{-}(CH_2)_3CO_2H$$

(c) Réaction de couplage :

$$C_8H_{17}\text{-}C\equiv C\text{-}CH_2\text{-}C\equiv C\text{-}CH_2Br + H\text{-}C\equiv C\text{-}(CH_2)_3CO_2H$$

$$\Big\downarrow \begin{array}{l}(1)\ 2\acute{e}q\text{-}C_2H_5\ MgBr\\(2)\ Cu_2(CN)_2\end{array}$$

$$C_8H_{17}\text{—}(C\equiv C\text{-}CH_2)_3\text{—}(CH_2)_2\text{—}CO_2H.$$

Schéma réactionnel B

a) Synthèse de l'halogéno-1 tétradécadiyne-2,5

$$\underset{(1)}{C_8H_{17}\text{-}C\equiv C\text{-}H} \xrightarrow[\underset{(2)}{(b)\ XCH_2C\equiv C\text{-}CH_2X}]{(a)\ base\ forte} \underset{(3)}{C_8H_{17}\text{-}C\equiv C\text{-}CH_2\text{-}C\equiv C\text{-}CH_2\text{-}X}$$

(b) Synthèse de l'acide hexyne-5 carboxylique

$$H\text{-}C{\equiv}C\text{-}(CH_2)_3\text{-}X \xrightarrow{KCN} H\text{-}C{\equiv}C\text{-}(CH_2)_3\text{-}CN$$

(4) (5)

$$\xrightarrow[(b)\ HCl]{(a)\ KOH} H\text{-}C{\equiv}C\text{-}(CH_2)_3\text{-}CO_2H$$

(6)

(c) Réaction de couplage

$$C_8H_{17}\text{-}C{\equiv}C\text{-}CH_2\text{-}C{\equiv}C\text{-}CH_2X + H\text{-}C{\equiv}C\text{-}(CH_2)_3\text{-}CO_2H$$

(3) (6)

2éq.-$C_2H_5$ MgBr
THF
$Cu_2(CN)_2$

$$C_8H_{17}\text{-}(C{\equiv}C\text{-}CH_2)_3\text{-}(CH_2)_2\text{-}CO_2H$$

(7)

La synthèse de l'acide hexyne-5 carboxylique (6) amené à réagir avec l'halogéno-1 tetradécadiyne-2,5 (3) est connue en elle-même et consiste, en un premier temps, à faire réagir le cyanure de potassium sur l'halogéno-1 pentyne (4). L'hexyne-5 nitrile (5) est alors transformé en acide correspondant (6) par action de la potasse suivi d'une acidification du milieu réactionnel.

L'acétylure de l'acide (6) est formé par réaction avec deux équivalents d'organomagnésien. Le dianion est alors couplé avec l'halogéno-1 tétradécadiyne dans un éther tel que le tétrahydrofuranne. Cette réaction peut éventuellement s'effectuer en présence d'un solvant aprotique tel que l'hexaméthylphosphoramide (HMPA). On obtient alors un bon rendement d'acide eicosatriynoïque-5,8,11 (7).

Les esters conformes à l'invention sont obtenus par des réactions connues en soi. Ils sont notamment préparés selon une première méthode par réaction sur l'acide de formule (7) du pentachlorure de phosphore, suivi de la réaction du chlorure d'acide correspondant (8) avec un alcool (9) (R'-OH), R' est un groupement alkyle inférieur en $C_1$-$C_8$ ou cycloalkyle en $C_4$-$C_6$, substitués par un ou plusieurs groupements hydroxyles et/ou interrompus par un ou plusieurs hétéroatomes choisis parmi l'oxygène et le soufre. Cette réaction correspond au schéma suivant :

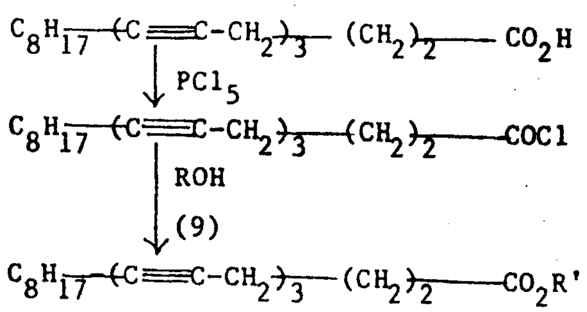


(7)

(8)

(I)

La deuxième méthode utilise le procédé décrit dans H.NORMANT et Coll., Synthesis 1975, page 805, qui consiste à former le sel de potassium de l'acide dans le DMF par réaction avec le bicarbonate de potassium en présence d'une diamine (N,N,N',N'-tetraméthylpropylène-diamine-1,3). On fait réagir ensuite le carboxylate de potassium sur un halogénure de formule (R'-X) où R' est tel que défini ci-dessus et X désigne halogène tel que Br ou Cl :

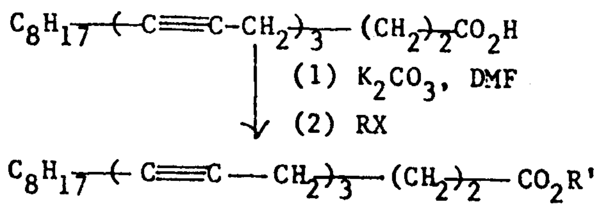


(7)

(I)

Lorsque la première méthode est utilisée et que le radical R' est substitué par au moins deux fonctions hydroxyles, comme par exemple l'ester de la glycérine, il est préférable de protéger deux des trois fonctions hydroxyles sous forme de dioxolane (11), selon le schéma réactionnel suivant :

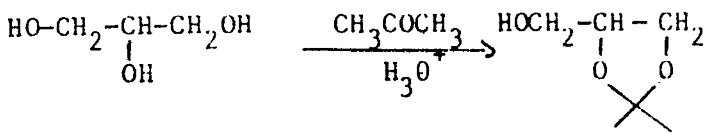


(11)

Le dioxolane (11) est alors mis en réaction avec le chlorure d'acide (8). L'ester (12) ainsi obtenu est traité dans des conditions douces dans le méthanol en présence d'un catalyseur acide pour éliminer le groupe protecteur des deux fonctions alcool, selon le schéma réactionnel suivant :

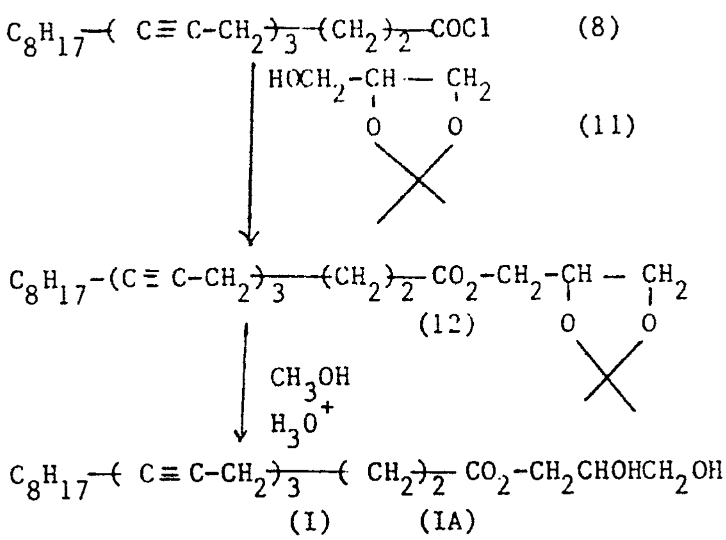


(8) (11) (12) (I) (IA)

et/ou 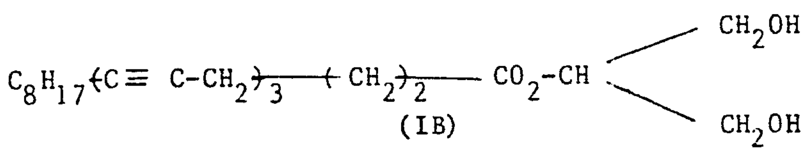

(IB)

Les amides conformes à l'invention peuvent être obtenus suivant deux méthodes.

La première méthode consiste à faire réagir le chlorure d'acide (8) sur l'amine 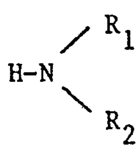 en présence d'une amine tertiaire selon le schéma réactionnel suivant :

(8) 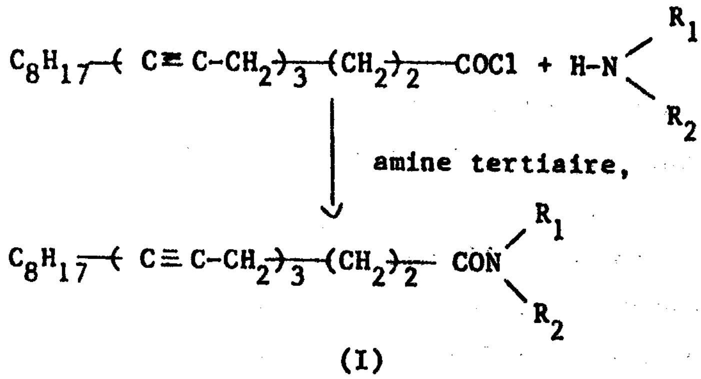


(I)

La seconde méthode met en oeuvre un procédé connu décrit dans (HEINZ A. STAAB, Liebigs Ann. Chem., 1957, 609, 75). Dans ce procédé, l'acide (7) est mis en réaction dans le DMF, porté à 80°C avec le carbonyldiimidazole (C.D.I.) pendant 2 à 3 heures. On ajoute ensuite à température ordinaire, un excès d'amine de formule 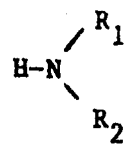 à l'intermédiaire formé selon le schéma réactionnel suivant :

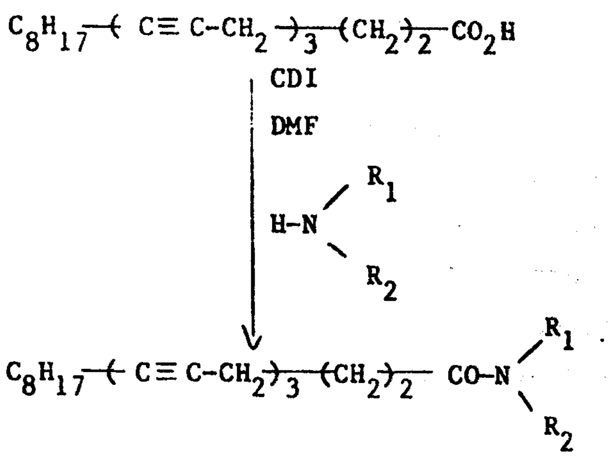


(7)

(I)

dans lesquelles $R_1$ et $R_2$ ont les significations indiquées ci-dessus.

Les composés de formule (I) conformes à l'invention ont une activité particulièrement remarquable vis-à-vis de l'inhibition du métabolisme de l'acide arachidonique et notamment au niveau des lipoxygénases à l'origine de la formation des leucotriènes et des acides hydroxylés qui jouent un rôle important dans le processus inflammatoire.

Les composés conformes à l'invention peuvent être administrés à l'homme ou à l'animal à l'aide de compositions contenant en plus un support ou diluant pharmaceutiquement acceptable. Ces compositions

peuvent également contenir éventuellement d'autres substances actives n'ayant pas d'effet antagoniste sur les composés conformes à l'invention.

Les compositions conformes à l'invention peuvent être administrées par voies systémique ou locale.

Pour la voie entérale, les médicaments peuvent se présenter sous forme de comprimés, de gélules, de dragées, de sirops, de suspensions, de solutions, de poudres, de granulés, d'émulsions etc. Pour la voie topique les compositions pharmaceutiques à base de composés conformes à l'invention se présentent sous forme d'onguents, de teintures, de crèmes, de pommades, de poudres, de timbres, de tampons imbibés, de solutions, de lotions, de gels, de sprays ou encore de suspensions.

Ces compositions pour la voie topique peuvent se présenter soit sous forme anhydre soit sous forme aqueuse selon l'indication clinique.

Les compositions conformes à l'invention peuvent également être administrées par voie parentérale et notamment par voie intraveineuse, intramusculaire, intrapéritonéale, sous-cutanée ou intradermique.

Pour les voies parentérales, etplus particulièrement les injections, on utilise la substance active dans un véhicule stérile tel que l'eau. La substance active est soit mise en suspension ou dissoute dans le véhicule.

Les composés conformes à l'invention peuvent également être utilisés en cosmétique notamment dans les crèmes, les lotions pour la peau telles que dans les produits solaires, post-solaires apaisants, antiséborrhéiques ou antiacnéiques.

Ces compositions médicamenteuses et cosmétiques conformes à l'invention peuvent contenir des additifs inertes ou pharmacodynamiquement ou cosmétiquement actifs et notamment :

des agents hydratants comme la thiamorpholinone et ses dérivés ou l'urée; des agents antiséborrhéiques, tels que la S-carboxyméthylcystéine, la S-benzylcystéamine et leurs dérivés, la tioxolone; des antibiotiques comme l'érythromycine, la néomycine ou les tétracyclines;

des agents favorisant la repousse des cheveux comme le "Minoxidil" (2,4-diamino 6-pipéridino pyrimidine 3-oxyde) et ses dérivés, le Diazoxyde et le Phénytoïn; d'autres agents anti-inflammatoires stéroïdiens ou non stéroïdiens; des caroténoïdes et notamment le $\beta$-carotène; des agents anti-psoriasiques tels que l'antraline et ses dérivés; des inhibiteurs de phospholipase $A_2$.

Ces compositions peuvent également contenir des agents d'amélioration de la saveur, des agents conservateurs, des agents stabilisants, des agents régulateurs d'humidité, des agents régulateurs de pH, des agents modificateurs de pression osmotique, des agents émulsionnants, des filtres UV-A et UV-B, des antioxydants tels que l'$\alpha$-tocophérol, le butylhydroxyanisole ou le butylhydroxytoluène, des anesthésiques locaux, des tampons etc.,

Elles peuvent être également conditionnées sous des formes retard ou à libération progressive connues en elles-mêmes. Elles peuvent enfin être introduites dans des phases aqueuses de liposomes ou de niosomes.

La substance active conforme à l'invention est présente dans les compositions dans des proportions comprises entre 0,01 et 10% en poids par rapport au poids total de la composition et de préférence entre 0,1 et 5%.

Dans l'application thérapeutique le traitement est déterminé par le médecin et peut varier suivant l'âge, le poids et la réponse du patient ainsi que la gravité des symptômes. Le dosage est généralement compris entre 0,05 et 300 mg/kg/jour et de préférence 0,5 à 100 mg/kg/jour. La durée du traitement est variable suivant la gravité des symptômes et peut s'étaler entre 1 et 12 semaines soit de façon continue ou discontinue.

Dans l'application cosmétique les compositions conformes à l'invention sont essentiellement utilisées comme produits solaires, apaisants post-solaires, et pour le traitement de dermatites séborrhéiques et/ou acnéiques.

Les exemples suivants sont destinés à illustrer l'invention sans pour autant présenter un caractère limitatif.

EXEMPLE DE REFERENCE

Préparation de l'acide eicosatriynoïque-5,8,11

a) Préparation du chloro-1 tétradécadiyne-2,5

Dans un ballon de 2 litres, muni d'une agitation, d'une arrivée d'argon, on place 19,35 g de magnésium (0,79 atome-gramme) puis 750 $cm^3$ de tétrahydrofuranne (THF) anhydre. On introduit alors goutte à goutte 90,25 g de bromure d'éthyle (1,15 équivalent) de façon à maintenir le T.H.F. sous reflux. A la fin de l'addition, ce dernier est maintenu jusqu'à transformation totale du magnésium. Ensuite, à cette température, on ajoute goutte à goutte, 100 g de décyne-1 (0,72 mole) en environ une demi-heure et le mélange est maintenu sous reflux encore une heure et demie après la fin de l'addition. On introduit alors 5,20 g de cyanure cuivreux et l'ébullition est maintenue encore une heure. A l'acétylure du décyne-1 ainsi formé, on ajoute à une température maintenue entre 40° et 50°C, 250 g de dichloro-1,4 butyne-2, soit 2,8 équivalents par rapport au décyne. L'ébullition est maintenue pendant 5 heures. Le solvant est éliminé à l'évaporateur rotatif. La masse huileuse obtenue est refroidie et agitée en présence de 350 $cm^3$ d'eau saturée de chlorure d'ammonium, puis extraite 3 fois à l'éther. La phase éthérée est lavée deux fois à l'eau, séchée sur sulfate de magnésium puis concentrée à la pression normale et ensuite le reste d'éther est éliminé sous une pression de 26,6 x $10^2$ Pa. Le chloro-1 tétradécadiyne-2,5 brut est distillé à une température comprise entre 118°C et 125°C sous 2,6 Pa. Dans les dernières fractions entre 125°C et 130°C sous 4 Pa, on distille le reste de produit attendu en mélange avec le bromo-1 tétradécadiyne-2,5, qui se forme au cours de la réaction par échange du chlore par le brome.

On obtient 100 g de chloro-1 tétradécadiyne-2,5 contenant environ 3 à 5% de bromo-1 tétradécadiyne-2,5 déterminé par RMN du proton que l'on utilise ainsi à la dernière étape de couplage.

b) Préparation de l'acide hexyne-5 carboxylique

A une solution, placée sous atmosphère inerte, de 60,4 g de cyanure de sodium (1,23 mole) dans 200 $cm^3$ de diméthylsulfoxyde (DMSO)

anhydre, on introduit goutte à goutte, à une température comprise entre 40 et 50°C, une solution de 115 g de chloro-1 pentyne-4 (1,12 mole) dans 100 $cm^3$ de D.M.S.O. anhydre. La réaction est exothermique et il est nécessaire de refroidir le mélange réactionnel pour maintenir la température inférieure à 70°C. A la fin de l'addition, le mélange est porté pendant 5 heures entre 60° et 70°C, puis abandonné une nuit à 20°C. Il est alors versé sur 1,5 litre d'eau. Le mélange obtenu est extrait 4 fois par 200 $cm^3$ d'éther. Les phases éthérées sont rassemblées, puis lavées deux fois avec 60 $cm^3$ d'eau, et séchées sur du sulfate de sodium. L'éther est rectifié sous pression réduite. On obtient 101 g d'hexyne-5 nitrile-1. C'est un liquide jaune que l'on utilise directement pour l'étape suivante. Les spectres $^1H$ R.M.N. et I.R correspondent à la structure attendue.

On introduit 200 g de nitrile précédent (2,15 mole) dans 2,5 litres de potasse 2N. Ce mélange est agité pendant deux heures à 90°C, puis refroidi à 0°C; on ajoute alors de l'acide chlorhydrique 5N jusqu'à pH $\simeq$ 4 du mélange. Ce dernier est extrait quatre fois par 300 $cm^3$ d'éther. Les phases éthérées rassemblées, sont lavées deux fois avec une solution aqueuse de chlorure d'ammonium, puis séchées sur sulfate de magnésium et concentrées sous pression réduite.

On obtient 230 g d'acide hexyne-5 carboxylique. C'est un liquide à la température ordinaire qui cristallise à 0°C. Les spectres R.M.N. et I.R. correspondent à la structure attendue.

Analyse élémentaire : $C_6H_8O_2$

| | C | H | O |
|---|---|---|---|
| Calculé | 64,27 | 7,19 | 28,54 |
| Trouvé | 64,32 | 7,21 | 28,41 |

c) <u>Préparation de l'acide eicosatriynoïque-5,8,11</u>

Dans 200 $cm^3$ de T.H.F., on transforme comme précédemment 20,05 g de magnésium (0,835 atome-gramme) en bromure d'éthylmagnésium par addition de 91 g de bromure d'éthyle (0,835 mole). A une solution préparée séparément et contenant 46,64 g d'acide hexyne-5 carboxylique (0,417 mole) dans 200 $cm^3$ de T.H.F. anhydre, agitée à 0°C sous atmosphère inerte, on ajoute la solution de bromure d'éthylmagnésium préparée ci-dessus.

A la fin de l'addition, on ajoute 25 $cm^3$ d'hexaméthylphosphoramide, puis 2,5 g de cyanure cuivreux. Le mélange ainsi obtenu est alors porté pendant environ une heure à une température de 50°C pour s'assurer de la transformation totale de l'acide hexyne-5 carboxylique en acétylure correspondant sous forme de carboxylate de magnésium.

A ce mélange, on ajoute alors goutte à goutte, à une température comprise entre 40 et 50°C, une solution contenant 0,32 mole de chloro-1 tétradécadiyne dans 100 $cm^3$ de T.H.F.

Le milieu réactionnel est porté au reflux du T.H.F. pendant 21 heures. A ce stade, on ajoute 1,3 g de cyanure cuivreux supplémentaire et le reflux est encore maintenu pendant 8 heures supplémentaires. Le solvant est évaporé. Le liquide visqueux obtenu est acidifié par addition de 100 $cm^3$ d'acide sulfurique 4N, puis 100 $cm^3$ d'acide sulfurique 2N. Le milieu hétérogène obtenu est extrait trois fois par 250 $cm^3$ d'éther. Les phases éthérées sont rassemblées et lavées deux fois à l'aide de 150 $cm^3$ d'une solution aqueuse saturée de chlorure d'ammonium, décantées, séchées sur sulfate de sodium et concentrées. Le produit obtenu est repris par 100 $cm^3$ d'hexane et refroidi à 0°C.

Les cristaux sont essorés, lavés avec de l'hexane glacé et séchés. On obtient 48 g d'acide eicosatriynoïque-5,8,11 dont le point de fusion est de 69-70°C.

Les spectres $^1H$ R.M.N. et I.R. correspondent à la structure attendue.

EXEMPLE DE PREPARATION 1

Préparation du dihydroxy-2',3' propyl eicosatriynoate-5,8,11

a) Préparation du chlorure de l'acide eicosatriynoïque-5,8,11

A une solution de 4 g d'acide eicosatriynoïque-5,8,11 dans 50 $cm^3$ de chlorure de méthylène anhydre, agitée à 0°C, on ajoute lentement 4,1 g de pentachlorure de phosphore. Un quart d'heure plus tard, la solution est portée sous reflux pendant deux heures, temps au bout duquel la totalité de l'acide est transformée. Le solvant et l'oxychlorure de phosphore sont évaporés sous pression réduite. On obtient

ainsi le chlorure d'acide qui sera utilisé brut pour la suite des réactions.

b) Préparation de l'ester de l'alcool diméthyl-2,2-hydroxyméthyl-4 dioxolane-1,3

Le chlorure d'acide obtenu à l'étape a), solubilisé dans 30 $cm^3$ de chlorure de méthylène anhydre, est ajouté à un mélange de 2,5 $cm^3$ de pyridine, de 5 $cm^3$ de diméthyl-2,2 hydroxyméthyl-4 dioxolane-1,3 dans 50 $cm^3$ de chlorure de méthylène agité à 0°C sous atmosphère inerte. La solution obtenue est ensuite abandonnée pendant une nuit à la température ordinaire.

Le milieu réactionnel est lavé à l'eau, la phase organique est séchée sur sulfate de magnésium, puis déposée sur une colonne de chromatographie de gel de silice. L'ester attendu est élué avec un mélange acétate d'éthyle/hexane (1/9). Après concentration des phases d'élution, on isole 2,7 g d'ester sous forme d'un liquide visqueux dont les spectres $^1H$ R.M.N. et I.R. correspondent à la structure attendue.

c) Libération du groupe protecteur du groupement dihydroxy-2',3' propyle

Une solution de 2 g d'ester protégé précédent est agité à la température ordinaire dans 30 $cm^3$ de méthanol en présence de 2,2 g d'Amberlite (résine sulfonique $\overset{+}{H}$) pendant trois jours.

A ce stade on s'assure, par vérification sur chromatographie en couche mince, que la partie dioxolane-1,3 est transformée en diol correspondant. La résine est alors éliminée par filtration. Le méthanol est évaporé sous pression réduite, le liquide obtenu est solubilisé dans le minimum de chlorure de méthylène, et la solution obtenue déposée sur une colonne de gel de silice.

L'ester est élué au mélange acétate d'éthyle/hexane (75/25).

Après concentration des phases d'élution, on obtient 1,1 g de dihydroxy-2',3' propyl eicosatriynoate-5,8,11. Le pic parent (m/e:374) en spectrographie de masse correspond bien à la masse molaire (M=374) attendue. Les spectres R.M.N. 250 MHz $^1H$ et $^{13}C$ indiquent que ce produit contient environ 8% de son isomère dihydroxy-1',3' propyl-2' eicosatriynoate-5,8,11.

EXEMPLE DE PREPARATION 2

Préparation du N-(hydroxy-2 éthyloxyéthyl)eicosatriynamide-5,8,11 (1ère voie de synthèse)

A un mélange de 0,36 g de diglycolamine, de 0,23 $cm^3$ de triéthylamine dans 20 $cm^3$ de chlorure de méthylène, refroidi à 0°C sous atmosphère inerte, on ajoute lentement une solution de 1 g de chlorure de l'acide eicosatriynoïque. La transformation du chlorure d'acide, suivie par chromatographie en couche mince, est très rapide. Lorsque celle-ci est totale, le milieu réactionnel est versé dans 100 $cm^3$ d'eau glacée. La phase organique est décantée. La phase aqueuse est extraite de nouveau trois fois au chlorure de méthylène. Les phases de chlorure de méthylène sont rassemblées, lavées à l'eau saturée de chlorure d'ammonium, puis séchées sur sulfate de magnésium et concentrées. On isole ainsi 0,6 g d'amide que l'on purifie par passage sur colonne de gel de silice. L'amide est élué à l'acétate d'éthyle. Après évaporation de l'éluant, on obtient 0,4 g de N-(hydroxy-2 éthyloxyéthyl)eicosatriynamide-5,8,11 sous forme de cristaux beiges dont le point de fusion est de 60°C.

Les spectres $^{13}C$ 250 MHz, infrarouge et de masse correspondent à la structure attendue.

EXEMPLE DE PREPARATION 3

Préparation du N-(hydroxy-2 éthyloxyéthyl)eicosatriynamide-5,8,11 (2ème voie de synthèse)

On prépare une solution de 15 g d'acide eicosatriynoïque-5,8,11 dans 150 $cm^3$ de diméthylformamide anhydre agitée sous atmosphère d'argon à laquelle on ajoute, à la température ordinaire, 10,5 g de carbonyldiimidazole. Le mélange est alors porté pendant trois heures à une température comprise entre 70° et 80°C. Refroidi à 0°C, on ajoute alors, goutte à goutte, 10,5 g de diglycolamine.

Le mélange est agité encore un quart d'heure après la fin de l'addition puis abandonné une nuit à la température ordinaire et versé sur 300 g de glace. Le produit précipite. Il est filtré, lavé à l'eau et séché. Les 18 g d'amide sont alors recristallisés dans 150 $cm^3$ d'acétonitrile. On obtient 17 g de N-(hydroxy-2 éthyloxyéthyl)

eicosatriynamide-5,8,11 sous forme de cristaux blancs dont le point de fusion est de 63°C.

Analyse élémentaire : $C_{24}H_{37}NO_3$

| | C | H | N | O |
|---|---|---|---|---|
| Calculé | 74,37 | 9,62 | 3,61 | 12,38 |
| Trouvé | 74,38 | 9,84 | 3,86 | 12,54 |

EXEMPLE DE PREPARATION 4

Préparation du N-éthyl eicosatriynamide-5,8,11

Un mélange agité sous atmosphère d'argon, de 1 g d'acide eicosatriynoïque-5,8,11, de 0,7 g de carbonyldiimidazole dans 10 $cm^3$ de D.M.F. anhydre est porté pendant 3 heures à une température de 80°C.

Ensuite, à 0°C, on ajoute en une fois une solution aqueuse à 33% de N-éthylamine. Une heure plus tard, on vérifie que l'acide de départ est transformé entièrement en amide correspondant. Le milieu réactionnel est versé dans 100 $cm^3$ d'eau glacée. Le produit précipité est essoré, lavé à l'eau et séché. On obtient 1,1 g de solide que l'on recristallise dans 10 $cm^3$ d'acétonitrile glacé.

On isole ainsi 0,85 g de N-éthyl eicosatriynamide-5,8,11 sous forme de cristaux blancs dont le point de fusion est de 68°C.

Analyse élémentaire : $C_{22}H_{33}NO$

| | C | H | N | O |
|---|---|---|---|---|
| Calculé | 80,68 | 10,15 | 4,27 | 4,88 |
| Trouvé | 80,82 | 10,37 | 4,21 | 5,10 |

EXEMPLE DE PREPARATION 5

Préparation du pyrrolidino eicosatriynamide-5,8,11

A une solution de 1 g d'acide eicosatriynoïque-5,8,11 dans 10 $cm^3$ de D.M.F. anhydre, agitée sous atmosphère d'argon, on ajoute, en une fois, 0,7 g de carbonyldiimidazole. La solution est alors portée trois heures à 80°C. Ensuite, à 0°C, on introduit 0,5 g de pyrrolidine. Deux heures après, la réaction est terminée.

Le mélange réactionnel est alors versé dans 100 $cm^3$ d'eau glacée, puis extrait au chlorure de méthylène. La phase organique est lavée à l'aide d'une solution d'acide chlorhydrique, puis à l'eau jusqu'à pH neutre, séchée sur sulfate de sodium et concentrée. On

obtient 1,1 g de pyrrolidino eicosatriynamide-5,8,11 sous forme d'un liquide jaune.

Les spectres I.R. et R.M.N. $^1H$ 250 MHz sont en accord avec la structure attendue.

L'analyse élémentaire correspond à un hémi-hydrate :

$C_{24}H_{35}NO,1/_2H_2O$.

| | C | H | N |
|---|---|---|---|
| Calculé | 79,50 | 10,00 | 3,86 |
| Trouvé | 79,22 | 9,38 | 4,00 |

## EXEMPLE DE PREPARATION 6

### Préparation du N,N-di(hydroxy-2 éthyl)eicosatriynamide-5,8,11

On traite l'intermédiaire formé à partir de 1 g d'acide et 0,7 g de carbonyldiimidazole dans 10 $cm^3$ de D.M.F. anhydre par 1 g de diéthanolamine.

Lorsque tout l'acide est transformé, le diméthylformamide est évaporé sous pression réduite. Le liquide obtenu est solubilisé au chlorure de méthylène, lavé en milieu acide et la phase organique est séchée sur sulfate de sodium puis agitée en présence de 15 g de gel de silice. Ce gel sur lequel s'est fixé l'amide est filtré. L'amide est alors libérée par extraction de la silice à l'acétate d'éthyle.

Après évaporation de ce solvant, on obtient 0,8 g de N,N-di(hydroxy-2 éthyl) eicosatriynamide-5,8,11. A la température ordinaire, c'est un liquide jaune dont les spectres I.R. et R.M.N. $^1H$ 250 MHz sont conformes à la structure attendue.

## EXEMPLE DE PREPARATION 7

### Préparation du N-(hydroxy-2 éthyl)eicosatriynamide-5,8,11

Une solution de 8 g d'acide eicosatriynoïque-5,8,11, de 5,62 g de carbonyldiimidazole dans 40 $cm^3$ de D.M.F. anhydre est portée à 80°C pendant 3 heures sous atmosphère inerte. Ensuite, à 0°C, on y ajoute 3,25 g d'éthanolamine. Par chromatographie en couche mince, 2 heures après, on vérifie que l'acide de départ est entièrement transformé.

Le mélange réactionnel est alors concentré par évaporation sous vide, puis dissous dans 100 $cm^3$ de chlorure de méthylène. La solution est lavée à l'eau, séchée sur sulfate de sodium et le chlorure de méthylène éliminé par évaporation sous vide. On obtient 9,1 g de solide. Après recristallisation de ce dernier, 8 g de N-(hydroxy-2 éthyl)eicosatriynamide-5,8,11 sont isolés sous forme de cristaux blancs dont le point de fusion est de 87°C.

Les spectres I.R. et R.M.N. $^1H$ 250 MHz correspondent à la structure attendue.

Analyse : $C_{22}H_{33}NO_2$

| | C | H | N | O |
|---|---|---|---|---|
| Calculé | 76,92 | 9,68 | 4,01 | 9,31 |
| Trouvé | 77,04 | 9,73 | 4,11 | 9,50 |

## EXEMPLE DE PREPARATION 8

Préparation de la N-(dihydroxy-2,3 propyl)eicosatriynamide-5,8,11

De la même façon que dans l'exemple précédent, 8 g d'acide eicosatriynoïque-5,8,11 dans 40 $cm^3$ de D.M.F. anhydre sont traités dans un premier temps par 5,62 g de carbonyldiimidazole. Ensuite, à 0°C, on ajoute 4,1 $cm^3$ de dihydroxy-2,3 propylamine. A la fin de la réaction, le mélange réactionnel est versé dans de l'eau glacée. Le produit précipité est essoré, dissous dans 300 $cm^3$ de chlorure de méthylène. La solution est séchée sur sulfate de sodium, puis concentrée sous pression réduite. Le solide obtenu est directement recristallisé dans l'acétonitrile. On isole ainsi 9 g de N-(dihydroxy-2,3 propyl)eicosatriynamide-5,8,11 sous forme de cristaux beige très clair de point de fusion de 87°C.

Les spectres I.R. et R.M.N. $^1H$ 250 MHz sont conformes à la structure attendue.

Analyse élémentaire : $C_{23}H_{35}NO_3$

| | C | H | N | O |
|---|---|---|---|---|
| Calculé | 73,95 | 9,44 | 3,75 | 12,85 |
| Trouvé | 73,85 | 9,50 | 3,93 | 12,65 |

EXEMPLE DE PREPARATION 9

Préparation de l'(hydroxy-2 éthyl)-4' pipérazino eicosatriynamide-5,8,11

Un mélange de 1 g d'acide eicosatriynoïque-5,8,11, de 0,7 g de carbonyldiimidazole (CDI) dans 5 $cm^3$ de D.M.F. anhydre, est agité sous atmosphère inerte pendant 3 heures à une température comprise entre 70-80°C. La solution est ensuite refroidie à 0°C, puis on ajoute une solution de 0,8 g d'(hydroxy-2 éthyl)-1 pipérazine dans 5 $cm^3$ de D.M.F. On maintient l'agitation pendant 2 heures et le milieu réactionnel est abandonné pendant la nuit. Le solvant est évaporé sous pression réduite. Le liquide obtenu est repris au chlorure de méthylène et la solution est lavée plusieurs fois à l'eau, séchée sur sulfate de sodium et concentrée sous pression réduite. On obtient 0,8 g d'(hydroxy-2 éthyl)-4' pipérazino eicosatriynamide-5,8,11, sous forme d'un liquide jaune.

Les spectres I.R. et $^1H$ 250 MHz correspondent à la structure attendue. L'analyse élémentaire correspond à un produit partiellement hydraté.

Analyse élémentaire : $C_{26}H_{40}N_2O_2$, 1/4 $H_2O$

| | C | H | N | O |
|---|---|---|---|---|
| Calculé | 74,86 | 9,78 | 6,71 | 8,63 |
| Trouvé | 74,42 | 10,42 | 6,72 | 8,49 |

## EXEMPLE DE PREPARATION 10

### Préparation du N-(para hydroxyphényl) eicosatriynamide-5,8,11

De la même façon qu'à l'exemple précédent on traite l'intermédiaire formé à partir de 1 g d'acide eicosatriynoïque-5,8,11 et de 0,7 g de C.D.I. par 0,727 g de para hydroxyaniline. Puis le milieu est agité 24 heures à la température ambiante. Ensuite, on ajoute progressivement de l'eau au mélange, jusqu'à ce que l'ensemble du produit formé précipite. Il est essoré et séché puis recristallisé dans l'éther isopropylique. On obtient 0,85 g de N-(parahydroxyphényl) eicostriynamide-5,8,11 sous forme de cristaux blanc-beige de point de fusion : 121°C.

Les spectres infrarouge et $^{1}H$ RMN 250 MHz sont conformes à la structure attendue.

Analyse élémentaire : $C_{26}H_{33}NO_2$

| | C | H | N | O |
|---|---|---|---|---|
| Calculé | 79,75 | 8,49 | 3,57 | 8,17 |
| Trouvé | 79,68 | 8,51 | 3,70 | 8,35 |

Les exemples suivants sont destinés à illustrer des compositions conformes à l'invention.

EXEMPLE 1

On prépare la composition suivante :

| | |
|---|---|
| - Dihydroxy-2',3'-propyl eicosatriynoate-5,8,11 | 0,50 g |
| - Propanol-1 | 50,00 g |
| - Propylèneglycol | 10,00 g |
| - Hydroxypropyl cellulose | 2,00 g |
| - Eau q.s.p. | 100 g |

Cette composition se présente sous forme d'un gel pouvant être appliqué par voie topique. On obtient également de bons résultats en remplaçant dans la composition le dihydroxy-2',3'-propyl eicosatriynoate-5,8,11 par le N-(hydroxy-2 éthyloxyéthyl) eicosatriynamide-5,8,11.

EXEMPLE 2

On prépare la composition suivante :

| | |
|---|---|
| - N-(hydroxy-2-éthyl) eicosatriynamide-5,8,11 | 5,0 g |
| - Polyéthylène micronisé | 10,00 g |
| - Myristate d'isopropyle q.s.p. | 100 g |

Cette composition se présente sous forme d'un onguent hydrophobe destiné à une application topique. On obtient également de bons résultats en remplaçant dans cet onguent le N-(hydroxy-2-éthyl) eicosatriynamide-5,8,11 par le N-(dihydroxy-2,3-propyl) eicosatriynamide-5,8,11 ou encore par le (hydroxy-2 éthyloxyéthyl) eicosatriynamide-5,8,11.

EXEMPLE 3

On prépare la composition suivante :

| | |
|---|---|
| - N-éthyl eicosatriynamide-5,8,11 | 1,00 g |
| - Triglycérides des acides caprique, caprylique et stéarique | 40,00 g |
| - Triglycérides des acides caprique et caprylique | 30,00 g |
| - Vaseline | 20,00 g |
| - Huile de vaseline q.s.p. | 100 g |

Cette composition se présente sous forme d'un onguent hydrophobe destiné à une application topique.

Il est possible de remplacer dans cet onguent le N-éthyl eicosatriynamide-5,8,11 par le pyrrolidino eicosatriynamide-5,8,11.

EXEMPLE 4

On prépare la composition suivante :

| | |
|---|---|
| - (Hydroxy-2'-éthyl)-4'-pipérazino eicosatriynamide-5,8,11 | 0,50 g |
| - Alcool cétylique | 6,40 g |
| - Alcool cétylique oxyéthyléné à 20 moles d'oxyéthylène | 2,10 g |
| - Monostéarate de glycérol | 2,00 g |
| - Triglycérides des acides caprique et caprylique | 15,00 g |
| - Propylèneglycol | 10,00 g |
| - Eau q.s.p. | 100 g |

Cette composition se présente sous forme d'une crème destinée à une application topique.

EXEMPLE 5

On prépare la lotion suivante :

| | |
|---|---|
| - Dihydroxy-2',3'-propyl eicosatriynoate-5,8,11 | 0,10 g |
| - Ethanol | 50,00 g |
| - Propylèneglycol q.s.p.. | 100 g |

Cette lotion est utilisée pour une application topique.

Les compositions des exemples 1 à 5 qui précèdent sont toutes fabriquées et stockées en atmosphère inerte et à l'abri de la lumière.

EXEMPLE 6

On prépare la composition suivante :

| | |
|---|---|
| - Dihydroxy-2',3' propyl eicosatriynoate-5,8,11 | 0,01 g |
| - Ethanol absolu | 1,00 ml |
| - Aromatisant q.s., conservateur q.s., glycérol q.s.p. | 5,00 ml |

qui est introduite dans une ampoule en verre brun de 5 ml et destinée à être utilisée par voie orale sous forme d'un soluté buvable.

EXEMPLE 7

On prépare une gélule de 350 mg contenant une poudre ayant la composition suivante :

| | |
|---|---|
| - N-(hydroxy-2 éthyloxyéthyl) eicosatriynamide-5,8,11 | 0,025 g |
| - Cellulose microcristalline | 0,020 g |
| - Amidon de maïs | 0,100 g |
| - Silice colloïdale | 0,020 g |
| - Stéarate de magnésium | 0,185 g |

REVENDICATIONS

1. Composé caractérisé par le fait qu'il répond à la formule :

$$C_8H_{17}-(C\equiv C-CH_2)_3-CH_2CH_2COR \qquad (I)$$

dans laquelle R est un groupement alcoxy inférieur en $C_1$-$C_8$ ou cycloalcoxy en $C_4$-$C_6$, substitués par un ou plusieurs groupements hydroxyles et/ou interrompu par un ou plusieurs hétéroatomes choisis parmi l'oxygène et le soufre, un

groupement amino de structure 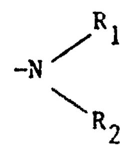 dans laquelle $R_1$ ou $R_2$ identiques ou différents désignent un atome d'hydrogène, un radical alkyle inférieur en $C_1$-$C_8$ linéaire ou ramifié, éventuellement interrompu par un ou plusieurs hétéroatomes choisis parmi l'oxygène, le soufre, l'azote, ce radical alkyle pouvant être substitué par un ou plusieurs groupements hydroxyles, $R_1$ et $R_2$ ne pouvant désigner simultanément hydrogène ou bien $R_1$ et $R_2$ forment ensemble avec l'atome d'azote, un hétérocycle contenant éventuellement comme hétéroatome supplémentaire de l'oxygène, du soufre ou de l'azote, l'un des radicaux $R_1$ ou $R_2$ pouvant encore désigner, lorsque l'autre est un atome d'hydrogène, un radical aryle de formule (II) :

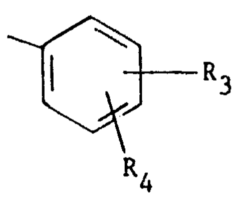

(II)

ou bien un radical benzyle de formule (III) :

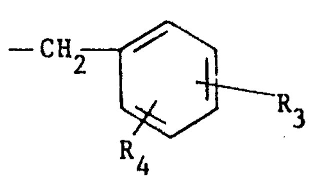

(III)

dans lesquelles $R_3$ et $R_4$ désignent, indépendamment l'un de l'autre un atome d'hydrogène, un radical alkyle en $C_1$-$C_4$, un groupement hydroxyle, un atome d'halogène, un groupement carboxyle ou trifluorométhyle, la fonction amine pouvant également provenir d'un sucre ainsi que leurs isomères et sels pharmaceutiquement et cosmétiquement acceptables.

2. Composé selon la revendication 1, caractérisé par le fait que le groupement COR dans la formule (I) est une fonction ester d'un glycol, d'un glycérol, d'un polyéthylèneglycol ou d'un sucre.

3. Composé selon la revendication 1, caractérisé par le fait que R a les significations suivantes :

$O-CH_2-CH_2-OH$, $O-CH_2-CHOH-CH_3$, $O-CH_2-CHOH-CH_2-OH$, 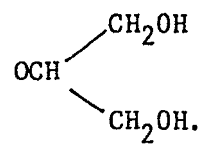

4. Composé selon la revendication 1, caractérisé par le fait que lorsque R désigne le groupement amino de formule 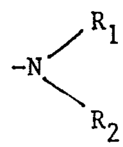 , $R_1$ et/ou $R_2$ désignent un radical alkyle inférieur en $C_1$-$C_4$, un radical alkyle inférieur interrompu par un atome d'oxygène et/ou substitué par un ou plusieurs groupements hydroxyles.

5. Composé selon la revendication 1, caractérisé par le fait que l'un au moins des groupements $R_1$ et $R_2$, désigne un radical alkyle en $C_1C_8$ éventuellement interrompu par un ou plusieurs atomes d'oxygène et substitué par un ou plusieurs groupements hydroxyle; ou bien que $R_1$ et $R_2$ forment avec l'atome d'azote un hétérocycle contenant éventuellement comme hétéro-atome supplémentaire un ou plusieurs atomes d'oxygène, de soufre ou d'azote.

6. Composé selon la revendication 5, caractérisé par le fait que les composés de formule (I) où R désigne le groupement

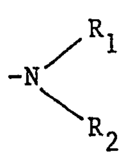 , $R_1$ et/ou $R_2$ sont choisis parmi les groupements suivants : $CH_2CH_2OCH_2CH_2OH$, $CH_2CH_2OH$, $CH_2CHOHCH_3$, $CH_2CHOHCH_2OH$.

7. Composé selon la revendication 5, caractérisé par le fait que $R_1$ et $R_2$ forment un cycle dérivé de la pyrrolidine, la morpholine, la pipérazine, l'hydroxy-2' éthyl-4 pipérazine.

8. Composé selon la revendication 1, caractérisé par le fait qu'il est choisi parmi le dihydroxy-2', 3' propyl eicosatriynoate-5,8, 11; le N-éthyl eicosatriynamide 5,8,11; le pyrrolidino eicosatriynamide-5,8,11; le N,N-di(hydroxy-2 éthyl)eicosatriynamide-5,8,11; le N-(hydroxy-2 éthyl)eicosatriynamide-5,8,11; le N-(dihydroxy-2,3 propyl)eicosatriynamide-5,8,11; le N-(parahydroxyphényl)eicosatriynamide-5,8,11.

9. Composé selon la revendication 5, caractérisé par le fait qu'il s'agit du N-(hydroxy-2 éthyloxyéthyl)eicosatriynamide-5,8,11.

10. Composé selon la revendication 5, caractérisé par le fait qu'il s'agit de 1'(hydroxy-2 éthyl)-4' pipérazino eicosatriynamide-5,8,11.

11. Procédé de préparation de l'acide eicosatriynoïque-5,8,11, caractérisé par le fait que l'on traite le décyne-1 répondant à la formule :

$C_8H_{17}$——— $C \equiv C-H$ (1)

avec une base forte pour former l'acétylure correspondant qui est amené à réagir avec le dihalogéno-1,4 butyne-2 :

$XCH_2C \equiv C-CH_2X$ (2)

pour obtenir l'halogéno-1 tétradécadiyne-2,5 de formule :

$C_8H_{17}-C \equiv C-CH_2-C \equiv C-CH_2X$ (3)

que l'on amène à réagir avec l'acide hexyne-5 carboxylique :

$H-C \equiv C\ (CH_2)_3COOH$ (6).

12. Procédé de préparation des composés selon l'une quelconque des revendications 1 à 10, dans lesquels COR est un ester, caractérisé par le fait que l'on transforme l'acide eicosatriynoïque-5,8,11 en chlorure d'acide correspondant sur lequel on fait réagir un alcool de formule R'-OH dans lequel R' désigne un groupement alkyle inférieur en $C_1$-$C_8$ ou cycloalkyle en $C_4$-$C_6$, substitués par un ou plusieurs groupements hydroxyle et/ou interrompus par un ou plusieurs hétéroatomes choisis parmi l'oxygène et le soufre, ou bien que l'on forme le sel de potassium de l'acide dans un solvant que l'on amène à faire réagir avec un halogénure de formule R'-X dans laquelle R' a la signification indiquée ci-dessus et X désigne chlore ou brome.

13. Procédé selon la revendication 12, caractérisé par le fait que lorsque l'alcool R-OH désigne un alcool comportant au moins trois groupements OH, on transforme au préalable l'alcool en dioxolane que l'on fait réagir avec le chlorure d'acide, les groupements protecteurs étant ensuite éliminés sur l'ester ou les isomères résultants.

14. Procédé de préparation des composés de formule (I) définis dans la revendication 1 dans lesquels COR est une fonction amide, caractérisé par le fait que l'on transforme l'acide eicosatriynoïque-5,8,11 en chlorure d'acide correspondant et que l'on fait réagir le chlorure d'acide sur une amine de formule 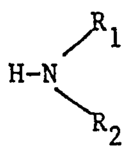 en présence d'une amine tertiaire, ou bien que l'on fait réagir sur l'acide eicosatriynoïque-5,8,11 le carbonyldiimidazole en présence d'un solvant et qu'on ajoute ensuite un excès d'amine de formule 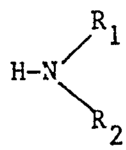 dans laquelle $R_1$ et $R_2$ ont les significations indiquées dans la revendication 1.

15. Médicament caractérisé par le fait qu'il contient un composé tel que défini dans l'une quelconque des revendications 1 à 10.

16. Composition pharmaceutique destinée à être administrée par voie systémique ou par voie locale, caractérisée par le fait qu'elle contient, en présence d'un support ou d'un diluant pharmaceutiquement acceptable, au moins un composé tel que défini dans l'une quelconque des revendications 1 à 10.

17. Composition pharmaceutique destinée à être administrée par voie topique, se présentant sous forme de crème, teinture, onguent, pommade, poudre, timbre, tampon imbibé, solution, gel, lotion, spray, suspension, caractérisée par le fait qu'elle contient au moins un composé tel que défini dans l'une des revendications 1 à 10.

18. Composition pharmaceutique se présentant sous forme de composition destinée à être administrée de façon parentérale par voie intraveineuse, intramusculaire, sous-cutanée, intradermique, caractérisée par le fait qu'elle contient un composé tel que défini dans l'une quelconque des revendications 1 à 10, dans un diluant pharmaceutiquement acceptable.

19. Composition pharmaceutique destinée à être administrée par voie entérale se présentant sous forme de comprimés, de gélules, de dragées, de sirops, de suspensions, de solutions, de poudres, de granulés, d'émulsions, caractérisée par le fait qu'elle contient un composé tel, que défini dans l'une quelconque des revendications 1 à 10.

20. Composition pharmaceutique selon l'une quelconque des revendications 16 à 19, caractérisée par le fait qu'elle contient le composé actif de formule (I) dans les proportions de 0,01 à 10% en poids par rapport au poids total de la composition et de préférence entre 0,1 et 5% en poids.

21. Utilisation d'un composé tel que défini dans l'une quelconque des revendications 1 à 10 pour la préparation d'un médicament destiné au traitement et à la prophylaxie des maladies allergiques et au traitement des dermatoses et des maladies inflammatoires telles que le psoriasis, l'eczéma, l'acné.

22. Composition cosmétique, caractérisée par le fait qu'elle contient au moins un composé tel que défini dans l'une quelconque des revendications 1 à 10 en une proportion comprise entre 0,01 et 10%, et de préférence, entre 0,1 et 5% en poids par rapport au poids total de la composition.

23. Application d'un composé tel que défini dans l'une quelconque des revendications 1 à 10 comme produit cosmétique.

( Autriche )

REVENDICATIONS

1. Procédé de préparation d'un composé répondant à la formule :

$$C_8H_{17}-(C\equiv C-CH_2)_3-CH_2CH_2COR \qquad (I)$$

dans laquelle R est un groupement alcoxy inférieur en $C_1$-$C_8$ ou cycloalcoxy en $C_4$-$C_6$, substitués par un ou plusieurs groupements hydroxyles et/ou interrompu par un ou plusieurs hétéroatomes choisis parmi l'oxygène et le soufre, un groupement amino de structure 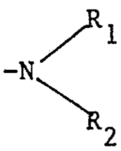 dans laquelle $R_1$ ou $R_2$ identiques ou différents désignent un atome d'hydrogène, un radical alkyle inférieur en $C_1$-$C_8$ linéaire ou ramifié, éventuellement interrompu par un ou plusieurs hétéroatomes choisis parmi l'oxygène, le soufre, l'azote, ce radical alkyle pouvant être substitué par un ou plusieurs groupements hydroxyles, $R_1$ et $R_2$ ne pouvant désigner simultanément hydrogène ou bien $R_1$ et $R_2$ forment ensemble avec l'atome d'azote, un hétérocycle contenant éventuellement comme hétéroatome supplémentaire de l'oxygène, du soufre ou de l'azote, l'un des radicaux $R_1$ ou $R_2$ pouvant encore désigner, lorsque l'autre est un atome d'hydrogène, un radical aryle de formule (II) :

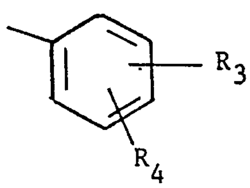

(II)

ou bien un radical benzyle de formule (III) :

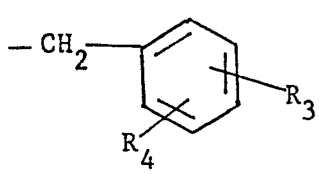

(III)

dans lesquelles $R_3$ et $R_4$ désignent, indépendamment l'un de l'autre un atome d'hydrogène, un radical alkyle en $C_1$-$C_4$, un groupement hydroxyle, un atome d'halogène, un groupement carboxyle ou trifluorométhyle, la fonction amine pouvant également provenir d'un sucre ainsi que

leurs isomères et sels pharmaceutiquement et cosmétiquement acceptables, caractérisé par le fait que

a) lorsque COR est un groupement ester l'on transforme l'acide eicosatriynoïque-5,8,11 en chlorure d'acide correspondant et qu'on fait réagir un alcool de formule R'-OH dans lequel R' est un groupement alkyle inférieur en $C_1$-$C_8$ ou cycloalkyle en $C_4$-$C_6$, substitués par un ou plusieurs groupements hydroxyle et/ou interrompus par un ou plusieurs hétéroatomes choisis parmi l'oxygène et le soufre ou bien que l'on forme le sel de potassium de l'acide dans un solvant que l'on amène à faire réagir avec un halogénure de formule R'-X dans laquelle R' a la signification indiquée ci-dessus et X désigne chlore ou brome,

b) lorsque COR est un groupement amide l'on transforme l'acide eicosatriynoïque-5,8,11 en chlorure d'acide correspondant et qu'on fait réagir le chlorure d'acide sur une amine

de formule 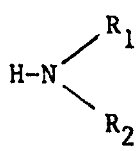 en présence d'une amine tertiaire, ou bien que l'on fait réagir sur l'acide eicosatriynoïque-5,8,11 le carbacarbonyldiimidazole en présence d'un solvant et qu'on ajoute ensuite un excès d'amine de formule 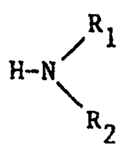 dans lesquelles $R_1$ et $R_2$ ont les significations indiqués ci-dessus.

2. Procédé selon la revendication 1, caractérisé par le fait que lorsque l'alcool R'-OH désigne un alcool comportant au moins trois groupements OH, on transforme au préalable l'alcool en dioxolane que l'on fait réagir avec le chlorure d'acide, les groupements protecteurs étant ensuite éliminés sur l'ester ou les isomères résultants.

3. Procédé de préparation selon la revendication 1, caractérisé par le fait que l'acide eicosatryinoïque-5,8,11 est préparé en traitant le décyne-1 répondant à la formule :

$C_8H_{17}$——$C\equiv C-H$ (1)

avec une base forte pour former l'acétylure correspondant qui est amené à réagir avec le dihalogéno-1,4 butyne-2 :

$XCH_2C\equiv C-CH_2X$ (2)

pour obtenir l'halogéno-1 tétradécadiyne-2,5 de formule :

$C_8H_{17}-C\equiv C-CH_2-C\equiv C-CH_2X$ (3)

que l'on amène à réagir avec l'acide hexyne-5 carboxylique :

$H-C\equiv C\ (CH_2)_3COOH$ (6).

4. Procédé de préparation d'une composition pharmaceutique destinée au traitement et à la prophylaxie de maladies allergiques et au traitement de dermatoses et des maladies inflammatoires telles que le psoriasis, l'eczéma, l'acné, caractérisé par le fait que l'on introduit dans un support ou diluant pharmaceutiquement acceptable 0,01 à 10% en poids par rapport au poids total de la composition du composé répondant à la formule :

$C_8H_{17}-(C\equiv C\ CH_2)_3-CH_2CH_2COR$ (I)

dans laquelle R est un groupement alcoxy inférieur en $C_1$-$C_8$ ou cycloalcoxy en $C_4$-$C_6$, substitués par un ou plusieurs groupements hydroxyles et/ou interrompu par un ou plusieurs hétéroatomes choisis parmi l'oxygène et le soufre, un

groupement amino de structure 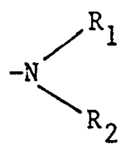 dans laquelle $R_1$ ou $R_2$ identiques ou différents désignent un atome d'hydrogène, un radical alkyle inférieur en $C_1$-$C_8$ linéaire ou ramifié, éventuellement interrompu par un ou plusieurs hétéroatomes choisis parmi l'oxygène, le soufre, l'azote, ce radical alkyle pouvant être substitué par un ou plusieurs groupements hydroxyles, $R_1$ et $R_2$ ne pouvant désigner simultanément hydrogène ou bien $R_1$ et $R_2$ forment ensemble avec l'atome d'azote, un hétérocycle contenant éventuellement comme hétéroatome supplémentaire de l'oxygène, du soufre ou de l'azote, l'un des radicaux $R_1$ ou $R_2$ pouvant encore désigner, lorsque l'autre est un atome d'hydrogène, un radical aryle de formule (II) :

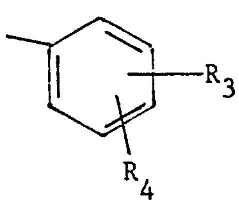

(II)

ou bien un radical benzyle de formule (III) :

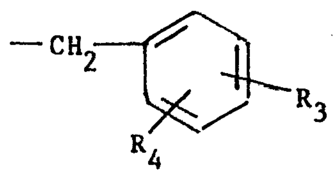

(III)

dans lesquelles $R_3$ et $R_4$ désignent, indépendamment l'un de l'autre un atome d'hydrogène, un radical alkyle en $C_1$-$C_4$, un groupement hydroxyle, un atome d'halogène, un groupement carboxyle ou trifluorométhyle, la fonction amine pouvant également provenir d'un sucre ainsi que leurs isomères et sels pharmaceutiquement et cosmétiquement acceptables.

5. Procédé selon la revendication 4, caractérisé par le fait que le groupement COR dans la formule (I) est une fonction ester d'un glycol, d'un glycérol, d'un polyéthylèneglycol ou d'un sucre.

6. Procédé selon la revendication 4, caractérisé par le fait que R a les significations suivantes :

$O-CH_2-CH_2-OH$, $O-CH_2-CHOH-CH_3$, $O-CH_2-CHOH-CH_2-OH$, 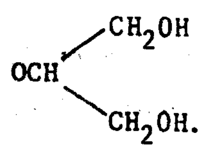.

7. Composé selon la revendication 4, caractérisé par le fait que lorsque R désigne le groupement amino de formule 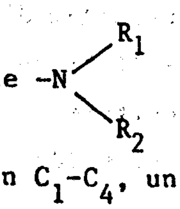, $R_1$ et/ou $R_2$ désignent un radical alkyle inférieur en $C_1$-$C_4$, un radical alkyle inférieur interrompu par un atome d'oxygène et/ou substitué par un ou plusieurs groupements hydroxyles.

8. Procédé selon la revendication 4, caractérisé par le fait que l'un au moins des groupements $R_1$ et $R_2$, désigne un radical alkyle en $C_1C_8$ éventuellement interrompu par un ou plusieurs atomes d'oxygène et substitué par un ou plusieurs groupements hydroxyle; ou bien que $R_1$ et $R_2$ forment avec l'atome d'azote un hétérocycle contenant éventuellement comme hétéro-atome supplémentaire un ou plusieurs atomes d'oxygène, de soufre ou d'azote.

9. Procédé selon la revendication 8, caractérisé par le fait que les composés de formule (I) où R désigne le groupement

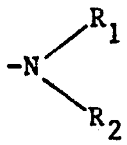

, $R_1$ et/ou $R_2$ sont choisis parmi les groupements suivants : $CH_2CH_2OCH_2CH_2OH$, $CH_2CH_2OH$, $CH_2CHOHCH_3$, $CH_2CHOHCH_2OH$.

10. Procédé selon la revendication 8, caractérisé par le fait que $R_1$ et $R_2$ forment un cycle dérivé de la pyrrolidine, la morpholine, la pipérazine, l'hydroxy-2' éthyl-4 pipérazine.

11. Procédé selon la revendication 4, caractérisé par le fait que le composé est choisi parmi le dihydroxy-2', 3' propyl eicosatriynoate-5,8, 11; le N-éthyl eicosatriynamide 5,8,11; le pyrrolidino eicosatriynamide-5,8,11; le N,N-di(hydroxy-2 éthyl)eicosatriynamide-5,8,11; le N-(hydroxy-2 éthyl)eicosatriynamide-5,8,11; le N-(dihydroxy-2,3 propyl)eicosatriynamide-5,8,11; le N(parahydroxyphényl)-eicosatriynamide-5,8,11.

12. Procédé selon la revendication 8, caractérisé par le fait que le composé est le N-(hydroxy-2 éthyloxyéthyl)eicosatriynamide-5,8,11.

13. Procédé selon la revendication 8, caractérisé par le fait que le composé est l'hydroxy-2 éthyl)-4' pipérazino eicosatriynamide-5,8,11.

14. Utilisation d'un composé tel que défini dans l'une quelconque des revendications 4 à 13 pour la préparation d'un médicament destiné au traitement et à la prophylaxie des maladies allergiques et au traitement des dermatoses et des maladies inflammatoires telles que le psoriasis, l'eczéma, l'acné.

15. Composition cosmétique, caractérisée par le fait qu'elle contient au moins un composé tel que défini dans l'une quelconque des revendications 4 à 13 en une proportion comprise entre 0,01 et 10%, et de préférence, entre 0,1 et 5% en poids par rapport au poids total de la composition.

16. Application d'un composé tel que défini dans l'une quelconque des revendications 4 à 13 comme produit cosmétique.

Office européen des brevets

**0209770**

Numero de la demande

# RAPPORT DE RECHERCHE EUROPEENNE

EP 86 10 9150

## DOCUMENTS CONSIDERES COMME PERTINENTS

| Catégorie | Citation du document avec indication, en cas de besoin des parties pertinentes | Revendication concernée | CLASSEMENT DE LA DEMANDE (Int Cl 4) |
|---|---|---|---|
| A | EP-A-0 104 468 (SYNTEX)<br>* Revendications *<br>--- | 1-23 | C 07 C 69/606<br>C 07 C 103/58<br>C 07 C 103/60<br>C 07 D 295/18<br>A 61 K 31/23<br>A 61 K 31/16<br>A 61 K 31/40 |
| D,A | US-A-4 190 669 (J.J. VOORHEES)<br>* Revendications *<br>----- | 1-23 | |

| DOMAINES TECHNIQUES RECHERCHES (Int Cl.4) |
|---|
| C 07 C 69/00<br>C 07 C 103/00<br>C 07 D 295/00<br>A 61 K 31/00 |

Le présent rapport de recherche a été établi pour toutes les revendications

| Lieu de la recherche | Date d'achèvement de la recherche | Examinateur |
|---|---|---|
| LA HAYE | 21-10-1986 | PAUWELS G.R.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant